# EUROPEAN PATENT APPLICATION

(11) **EP 1 949 862 A1**
(43) Date of publication of application: **30.07.2008**
(21) Application number: 06811871.0
(22) Date of filing: 17.10.2006
(51) Int. Cl.: A61B 17/221, A61B 17/00, A61B 17/50

(54) **GRASPING TOOL**

(30) Priority: 17.10.2005 JP 2005301444
(71) Applicant: JMS Co., Ltd., Hiroshima-shi, Hiroshima 730-8652 (JP)
(72) Inventor: KUMATE, Munetaka, Kurume-shi, Fukuoka 830-0038 (JP); HAYASHI, Shuro, Hiroshima-shi, Hiroshima 730-8652 (JP)
(74) Representative: Collin, Jérôme
(86) International application number: PCT/JP2006/320616
(87) International publication number: WO 2007/046355

(57) **Abstract**

A wire is folded at the central part in the longitudinal direction thereof to form a holding member including a first linear portion **17**, a return portion **18**, and a second linear portion **19**. The first linear portion **17** is inserted through a first pipe **13** of a guide member **2** while the second linear portion **19** is inserted through a second pipe **14** thereof. A first operation part **20** and a second operation part **23** are provided at the first linear portion **17** and the second linear portion **19**, respectively. The first linear portion **17** and the second linear portion **19** of the holding member **4** are protruded from the distal end of the guide tube **10** to form a loop **3**. The second linear portion **19** is protruded from the distal end of the guide tube **10** longer than the first linear portion **17** to change the shape of the loop **3** and to enlarge the loop **3**.

## Description

### Technical Field

Conventionally, as a holder of this kind, there has been known a holder including a long tubular guide member to be inserted in an organism, a flexible loop for holding a target object, such as a tissue, an indweller, or the like in the organism, and an operation wire for operating the loop (see Patent Document 1, for example). The operation wire is capable of being inserted in the guide member. The loop is formed along a plane, and one end of the operation wire is connected to one end of the loop.

For using the above holder, the operation wire is first operated to accommodate the loop into the guide member from one end of the guide member. Under this state, the guide member is inserted from the one end part thereof up to the vicinity of a target object in an organism, and then, the operation wire is operated to protrude out the loop accommodated in the guide member from the one end of the guide member. When the loop is moved within the organism by operating the operation wire to position the target object within the loop and is pulled into the guide member, a part on the operation wire side of the loop is gradually accommodated into the guide member to reduced the diameter of the loop, thereby allowing the loop to hold the target object.
Patent Document 1: Japanese Unexamined Patent Application Publication 2000-271146

### Disclosure of the Invention

### Problems that the Invention is to Solve

The inside of the organism is so complicated that it is difficult in some cases to position the guide member at the vicinity of the target object for positioning, as in the Patent Document 1, the loop at the vicinity of the target object by inserting the guide member into the organism. When the guide member is difficult to be positioned at the vicinity of the target object, it is necessary to operate the operation wire for positioning the loop at the vicinity of the target object.

In the holder in Patent Document 1, however, since the loop is merely formed along a plane and connected to one end of the operation wire, the shape of the loop when completely protruded out from the guide member is substantially fixed. For this reason, the shape and the size of the loop cannot be changed to those allowing the loop to hold the target object easily. Further, in the case where the holder holds a target object within a blood vessel, for example, the loop may move or change in shape by the bloodstream in the blood vessel even with the operation wire not operate to make it difficult to hold the target object.

In short, it is difficult for the holder in Patent Document 1 to hold a target object when it is difficult to insert the guide member up to the vicinity of the target object or when the target object is to be held within a blood vessel, so that it takes much time for treatment, thereby invading a patient severely.

The present invention has been made in view of the foregoing and has its object of reducing invasion on a patient by changing the shape and the size of a loop within an organism for allowing the loop to hold a target object readily.

### Means for Solving the Problems

In order to achieve the above object, in the present invention, a loop is formed of a holding member including a first linear portion, a second linear portion, and a return portion continuing from each one end of the linear portions and the first linear portion and the second linear portion are made operable from the outside of an organism.

Specifically, in a first aspect of the invention, a holder includes: a long tubular guide member to be inserted at one end part thereof into an organism and to be protruded at the other end part thereof from the organism; and a long holding member of which one end part is inserted in the guide member to be guided into the organism and which forms a loop for holding a target object within the organism, wherein the holding member includes: a first linear portion extending from the one end part of the holding member and protruded from the other end part of the guide member; a return portion continuing from one end part of the first linear portion and returned toward the other end part of the guide member; and a second linear portion continuing from the return portion and extending toward and protruded from the other end part of the guide member, and a first operation part and a second operation part which are to be held and operated by an operator are respectively provided at parts of the first linear portion and the second linear portion which are protruded from the other end part of the guide member.

In the above arrangement, when the first operation part and the second operation part are operated to protrude the one end part of the holding member from the one end part of the guide member with the one end part of the guide member inserted within the organism, the loop is formed by the return portion and each one end part of the linear portions of the holding member. In the case where the it is difficult to insert the one end part of the guide member up to the vicinity of the target object within the organism, the first operation part and the second operation part are moved to protrude the linear portions long from the one end part of the guide member, thereby allowing the loop to approach the target object and being large. When only the second operation part is moved without moving the first operation part with the linear portions protruded from the one end part of the guide member to protrude the second linear portion long from the one end part of the guide member, the second linear portion curves away from the first linear portion to change its shape and be large because the linear portions are connected to each other via the return portion. In reverse, when only the first operation part is moved to protrude the first linear portion long from the one end part of the guide member, the first linear portion changes its shape and becomes large. Further, when the second operation is twisted without moving the first operation part, the twisting force is transmitted from the one end part of the second linear portion to the return portion to twist the loop entirely, thereby allowing the loop to be in a three-dimensional shape. Similarly, when only the first operation part is twisted, the loop is in a three-dimensional shape. Twisted operation of the first operation part and the second operation part changes the direction of the loop.

In short, operation of the first operation part and the second operation part outside the organism with the guide member and the one end part of the holding member inserted within the organism can change the position, the shape, the size, and the direction of the loop.

Referring to a second aspect of the present invention, in the first aspect, the other end part of the guide member branches into a first pipe through which the other end part of the first linear portion is inserted and a second pipe through which the other end part of the second linear portion is inserted.

In the above arrangement, the other end part of the first linear portion and the other end part of the second linear portion are protruded from the pipes different from each other. This enables separation of the first operation part and the second operation part from each other.

Referring to a third aspect of the present invention, in the second aspect, the first pipe is provided with first fixing means for switching the first linear portion to and from a state that the other end part thereof is fixed to the first pipe from and to a state that the fixed state is released.

With the above arrangement, in the case where it is desired to change the shape, the size, or the like of the loop slightly by operating only the second operation part, the first linear portion can be fixed to the first pipe by the first fixing means to be immovable.

Referring to a fourth aspect of the present invention, in the third aspect, the second pipe is provided with second fixing means for switching the second linear part to and from a state that the other end part thereof is fixed to the second pipe from and to a state that the fixed state is released.

With the above arrangement, in the case where it is desired to change the shape, the size, or the like of the loop slightly by operating only the first operation part, the second linear portion can be set to be immovable.

Referring to a fifth aspect of the present invention, in the first aspect, the first operation part and the second operation part are provided with identification parts for identifying the operation parts.

With the above arrangement, the operator can identify the first operation part and the second operation part effortlessly and correctly, thereby enabling secured operation of a desired operation part.

Referring to a sixth aspect of the present invention, in the first aspect, the guide member is divided into an insertion part to be inserted into the organism and a protruded part to be protruded outside the organism, and the insertion part is softer than the protruded part.

With the above arrangement, the inserted part of the guide member in inserting the guide member into the organism deforms readily along the shape of the inside of the organism.

In a seventh aspect of the present invention, an aspirator for aspirating a liquid in the guide member is connected to the guide member.

Referring to an eighth aspect of the present invention, in the first aspect, an insufflator for infusing a chemical solution into the guide member is connected to the guide member.

Referring to a ninth aspect of the present invention, in the first aspect, the guide member is bent at a part on the one end part side thereof.

Referring to a tenth aspect of the present invention, in the first aspect, a mark made of an X-ray non-transmissive material is provided at the one end part of the holding member.

Referring to an eleventh aspect of the present invention, in the first aspect, the inside of the guide member is divided into a room through which the first linear portion of the holding member is inserted and a room through which the second linear portion thereof is inserted.

### Effect of the Invention

In the first aspect of the present invention, the holding member forming the loop for holding the target object within the organism includes the first linear portion, the second linear portion, and the return portion continuing from the linear portions and the operation parts for the operator are provided at the first linear portion and the second linear portion, so that the loop can be changed in position, shape, size, and direction easily from the outside of the organism. This attains easy holding of the target object even in the case where it is difficult to insert the guide member up to the vicinity of the target object within the organism or the case where the target object within a blood vessel is to be held, thereby reducing invasion on the patient.

In the second aspect of the present invention, the other end part of the guide member branches into the first pipe through which the first linear portion is inserted and the second pipe through which the second linear portion is inserted to thus separate the first operation part and the second operation part from each other. This facilitates independent operation of the two operation parts, thereby achieving favorable operability.

According to the third aspect of the present invention, the first fixing means fixes the first linear portion to the first pipe to facilitate slight change in shape, size, and the like of the loop by operation of only the second operation part.

According to the fourth aspect of the present invention, the second fixing means fixes the second linear portion to the second pipe to facilitate slight change in shape, size, and the like of the loop by operating only the first operation part.

In the fifth aspect of the present invention, the identification parts are provided at first operation part and the second operation part to achieve further favorable operability.

In the sixth aspect of the present invention, the guide member is divided into the insertion part to be inserted into the organism and the protrusion part to be protruded outside the organism and the insertion part is set softer than the protrusion part, thereby further reducing invasion on the organism.

In the seventh aspect of the present invention, the guide member is connected to the aspirator, thereby enabling aspiration of blood and the like in the guide member.

In the eighth aspect of the present invention, the insufflator for a chemical solution is connected to the guide member, so that an anesthetic, for example, can be infused into the organism through the guide member.

According to the ninth aspect of the present invention, change in direction of the one end part of the guide member readily changes the direction of the one end part of the holding member.

According to the tenth aspect of the present invention, under radiography with the one end part of the holding member inserted in the organism, the operator can recognize the position of the one end part of the holding member to enable safe operation of the holding member.

In the eleventh aspect of the present invention, the inside of the guide member is divided into the room through which the first linear member is inserted and the room through which the second linear portion is inserted, so that one of the linear portions is prevented from obstructing the other linear portion in moving the other linear portion, thereby facilitating the operation.

### Brief Description of the Drawings

[FIG.1] FIG. **1** is a side view of a holder in accordance with an embodiment of the present invention.
[FIG. 2] FIG. **2** is a sectional view showing a construction of a first fixing mechanism.
[FIG. 3] FIG. **3** is a diagram for explaining a scheme for holding a stent within a blood vessel.
[FIG. 4] FIG. **4(a)** is a view showing a state where a holding member is protruded short from a guide tube; FIG. **4(b)** is a view showing a state where the holding member is protruded long form the guide tube; FIG. **4(c)** is a view showing a state where a second linear portion is protruded longer than a first linear portion; and FIG. **4(d)** is a view showing a state where the second linear portion is twisted when viewed from the distal end of the guide tube.
[FIG. **5**] FIG. **5** is a view corresponding to FIG. **4(b)** in accordance with Modified Example 1 of the embodiment.
[FIG. **6**] FIG. **6** is a view corresponding to FIG. **1** in accordance with Modified Example 2 of the embodiment.
[FIG. **7**] FIG. **7** relates to Modified Example 3 of the embodiment, wherein FIG. **7(a)** is a view corresponding to FIG. **4(b)** and FIG. **7(b)** is a sectional view taken along the line A-A in FIG. **7(a)**.
[FIG. **8**] FIG. **8** relates to Modified Example 4 of the embodiment, wherein FIG. **8(a)** is a view corresponding to FIG. **4(b)** and FIG. **8(b)** is a sectional view taken along the line B-B in FIG. **8(a)****.**
[FIG. **9**] FIG. **9** is a view corresponding to FIG. **4(b)** in accordance with Modified Example 5 of the embodiment.
[FIG. **10**] FIG. **10** is a view corresponding to FIG. **4(b)** in accordance with Modified Example 6 of the embodiment.

### Explanation of Reference Numerals

- **1**: holder
- **2**: guide member
- **4**: holding member
- **13**: fist pipe
- **14**: second pipe
- **17**: first linear portion
- **18**: return portion
- **19**: second linear portion
- **20**: first operation part
- **21**: first cylindrical part (identification part)
- **23**: second operation part
- **24**: second cylindrical part (identification part)
- **30**: first fixing mechanism (first fixing means)
- **41**: second fixing mechanism (second fixing means)
- **W**: target object

### Best Mode for Carrying out the Invention

An embodiment of the present invention will be described below in detail with reference to the accompanying drawings. The following description of the preferred embodiment is a mere substantial example and is not intended to limit the present invention, applicable objects, and usage thereof.

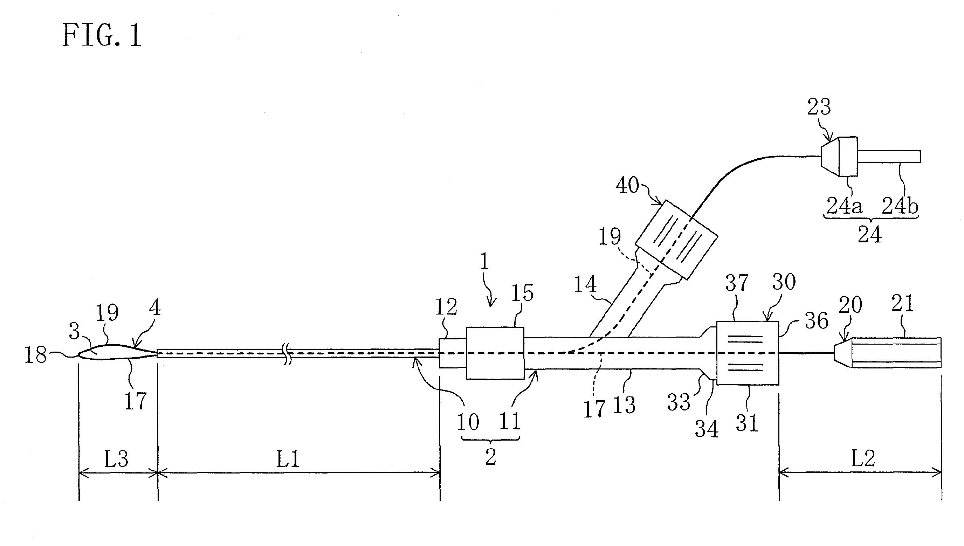

FIG. **1** shows a holder **1** in accordance with one embodiment of the present invention. The holder **1** is used for holding a target object within the body of a patient and taking it out from the body. The holder **1** includes a long tubular guide member **2** formed so as to be inserted at one end part thereof into the body and be protruded at the other end part thereof outside the body and a long holding member **4** forming a loop **3** for holding a target object within the body. The holding member **4** is inserted in the guide member **2** to be guided into the patient's body.

The guide member **2** includes a long and small-diameter guide tube **10** on the side to be inserted into the body and a branch pipe **11** on the side to be protruded from the body. The guide tube **10** is made of a soft resin material so as to be readily curved and deformed at insertion into the body. The length, the outer diameter, and the inner diameter of the guide tube **10** may be changed according to a part where the holder **1** is used, a kind of the target object, the build, the age and the sex of the patient, and the like. At the end on the branch pipe **11** side of the guide tube **10**, a tubular joint **12** is provided to be connected to the branch pipe **11**. A radially protruding and peripherally extending stripe protrusion (not shown) is formed at the outer periphery of the joint **12**.

The branch pipe **11** is in a Y shape of a first pipe **13** extending along the center line of the guide tube **10** and a second pipe **14** branching from the middle part in the longitudinal direction of the first pipe **13**. The first pipe **13** and the second pipe **14** are made of a resin material harder than the resin material of the guide tube **10** and is formed integrally with each other so as not to be readily deformed in operation. Openings are formed in the first pipe **13** and the second pipe **14** at the respective ends opposite to the guide tube **10**. The branch pipe **11** may be formed of a known Y-shaped connector.

At the end on the guide tube **10** side of the first pipe **13**, an expansion part **15** of which outer diameter expands is provided. An engaging groove (not shown) for receiving the stripe protrusion of the guide tube **10** is formed in the inner face of the expansion part **15**. When the stripe protrusion is fitted into the engaging groove, the guide tube **10** and the branch pipe **11** are integrated with each other. Accordingly, the guide tube **10** is exchangeable with another guide tube **10** different in length or the like.

The holding member **4** is a long medical wire with a line diameter in the range between 0.3 mm and 0.7 mm, both inclusive, which is folded around the central part in the longitudinal direction thereof. Specifically, the holding member **4** includes: a first linear portion **17** extending from the distal end of the guide tube **10** toward the branch pipe **11**; a return portion **18** continuing from the guide tube **10** side end of the first linear portion **17** and folded toward the branch pipe **11**; and a second linear portion **19** continuing from the return portion **18** and extending toward the branch pipe **11**. The first linear portion **17** and the second linear portion **19** are longer than the length along the center line of the guide member **2**. Accordingly, the branch pipe **11** side ends of the first linear portion **17** and the second linear portion 19 protrude outside the openings of the first pipe **13** and the second pipe **14**, respectively.

The return portion **18** is formed around the central part in the longitudinal direction of the wire of the holding member **4**, namely, is a folded part of the wire. The return portion **18** is so formed to urge the first linear portion **17** and the second linear portion **19** away from each other.

A part of the first linear portion **17** which is protruded from the first pipe **13** serves as a first operation part **20**. On the end of the first operation part **20** opposite to the first pipe **13**, a first cylindrical part **21** in a substantially cylindrical shape made of a resin material is detachably mounted. A central hole (not shown) is formed in the first cylindrical part **21** so that the first linear portion **17** is inserted therein and is fixed to the first cylindrical part **21**. Whereby, the first linear portion **17** is rotated integrally with the first cylindrical part **21** with it prevented from being pulled out from the central hole.

A portion of the second linear portion **17** which is protruded from the second pipe **14** serves as a second operation part **23**. On the end of the second operation part **23** opposite to the second pipe **14**, a second cylindrical part **24** in a substantially cylindrical shape made of a resin material is detachably mounted. The second cylindrical part **24** includes a large diameter part **24a** and a small diameter part **24b** so as to be different in shape from the first cylindrical part **21**. A central hole (not shown) is formed in the second cylindrical part **24** so that the second linear portion **19** is inserted therein and is fixed to the second cylindrical part **24**. Accordingly, the second linear portion **19** is rotated integrally with the second cylindrical part **24** with it prevented from being pulled out from the central hole. Further, the first cylindrical part **21** is different in color of the resin material from the second cylindrical part **24**. In other words, the first cylindrical part **21** and the second cylindrical part **24** form identification parts for identifying the first operation part **20** and the second operation part **23**.

As the holding member **4**, a plurality of holding members different in line diameter, strength, or the like from each other may be prepared. When prepared, one of the holding members **4** which is suitable for a patient can be selected and exchanged. For exchange, the first cylindrical part **21** and the second cylindrical part **24** are taken off from the first linear portion **17** and the second linear portion **19**, respectively, and the holding member **4** is pulled out from the distal end of the guide tube **10**. Then, another holding member **4** for exchange is inserted from the distal end of the guide tube **10**, and the first cylindrical part **21** and the second cylindrical part **24** are mounted to the first linear portion **17** and the second linear portion **19**, respectively.

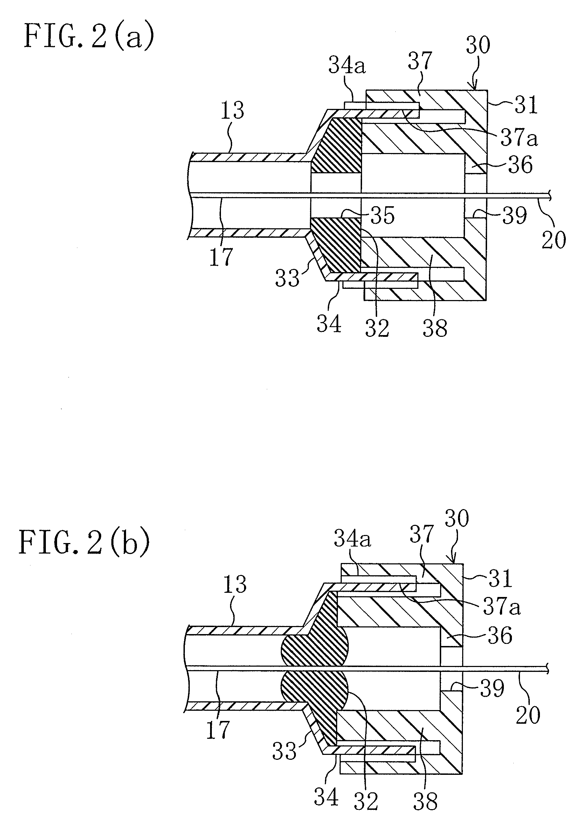

At the end of the first pipe **13** on the side opposite to the guide tube **10**, a first fixing mechanism **30** for fixing the first linear portion **17** to the first pipe **13** is provided. The first fixing mechanism **30** is composed of, as shown in FIG. **2**, a part of the first pipe **13** on the side opposite to the guide tube **10**, a cap member **31** covering the opening of the first pipe **13**, and an elastic member **32** inserted in the inside of the first pipe **13** on the side opposite to the guide tube **10**. At the part of the first pipe **13** on the side opposite to the guide tube **10**, there are provided a tapered face **33** formed so as to enlarge in diameter as it goes toward the end thereof and an annular part **34** continuing from the tapered face **33**. A male screw part **34a** is formed around the outer periphery of the annular part **34**.

The elastic member **32** is made of a readily deformed soft material, for example, silicone rubber or the like. The outer face of the elastic member **32** tightly adheres to the tapered face **33** and the inner face of the annular part **34**. In the elastic member **32**, a circular hole **35** with a diameter larger than the line diameter of the first linear portion **17** is formed coaxially with the center line of the first pipe **13**, and the first linear portion **17** is inserted through the circular hole **35**. The cap member **31** is made of a resin material similar to the material of the first pipe **13** and includes a disc part **36** covering the end part of the first pipe **13** and an outer peripheral wall **37** and an inner peripheral wall **38** which are protruded from the first pipe **13** side of the disc part **36**. An insertion hole **39** with a diameter larger than the line diameter of the first linear portion **17** is formed in the disc part **36** coaxially with the center line of the first pipe **13**. The outer peripheral wall **37** is formed along the outer face of the annular part **34**. A female screw part **37a** to be in screw engagement with the male screw part **34a** of the annular part **34** is formed in the inner face of the outer peripheral wall **37**. The inner peripheral wall **38** is separated inward from the inner face of the annular part **34**, and the distal end in the protruding direction of the inner peripheral wall **38** is in contact with the elastic member **32**.

When the female screw part **37a** of the cap member **31** is screwed into the male screw part **34a** of the first pipe **13**, the end part of the inner peripheral wall **38** of the cap member **38** pushes the elastic member **32** toward the tapered face **33**, so that the inner peripheral wall **38** approaches the tapered face **33** of the first pipe **13**. The elastic member **32** pushed by the inner peripheral wall **38** is elastically deformed to reduce the inner diameter of the circular hole **35**, so that the inner face of the circular hole **35** adheres to the first linear portion **17** tightly. At this time point, the elastic member **32** is pushed against and adheres tightly to the tapered face **33**. Thus, the first linear portion **17** is in a state fixed to the fist pipe **13**.

In reverse, when the cap member **31** fixing the first linear portion **17** is screwed in the direction for detaching the cap member **17** from the first pipe **13**, the inner peripheral wall **38** separates from the tapered face **33** so that the elastic member **32** recovers to the original shape. This releases the fixed state of the first linear portion **17**.

A second fixing mechanism **40** similarly to the first fixing mechanism **30** is provided at the end of the second pipe **14** on the side opposite to the guide tube **10**. The second fixing mechanism **40** fixes the second linear portion **19** to the second pipe **14**. The first fixing mechanism **30** and the second fixing mechanism **40** serve first fixing means and second fixing means, respectively, in the present invention.

As the holder **1**, child and adult holders each part of which is different in size from each other are prepared. Referring first to the size of each part of the child holder **1**, the thickness of the guide tube **10** is set at approximately 3 to 4 Fr (french) so that the guide tube **10** can be easily inserted into a child's body. The length L1 (indicated in FIG. **1**) of the guide tube **10** is set at approximately 600 mm. The distance L2 from the first fixing mechanism **30** to the base end of the first cylindrical part **21** is set at approximately 200 mm, and the distance from the second fixing mechanism **40** to the base end of the second cylindrical part **24** is set substantially the same. The length L3 of the loop **3** protruding from the distal end of the guide tube **10** is set at approximately 100 mm. When the protruding length of the loop **3** is set at 100 mm or smaller, insertion into, for example, a heart can be carried out effortlessly.

On the other hand, the size of each part of the adult holder **1** is referred to. The thickness of the guide tube **10** is set at approximately 5 to 6 Fr (french), and the length thereof is set at approximately 1000 mm. The distance from the first fixing mechanism **30** to the base end of the first cylindrical part **21**, the distance from the second fixing mechanism **40** to the base end of the second cylindrical part **24**, and the length of the loop **3** protruding from the distal end of the guide tube **10** are set substantially the same as those set in the child holder **1**.

When the distance from the first fixing mechanism **30** to the base end of the first cylindrical part **21** and the distance from the second fixing mechanism **40** to the base end of the second cylindrical part **24** are set at approximately 200 mm or greater, the operator can operate the first cylindrical part **21** and the second cylindrical part **24** easily from the position apart from the fixed mechanisms **30**, **40**.

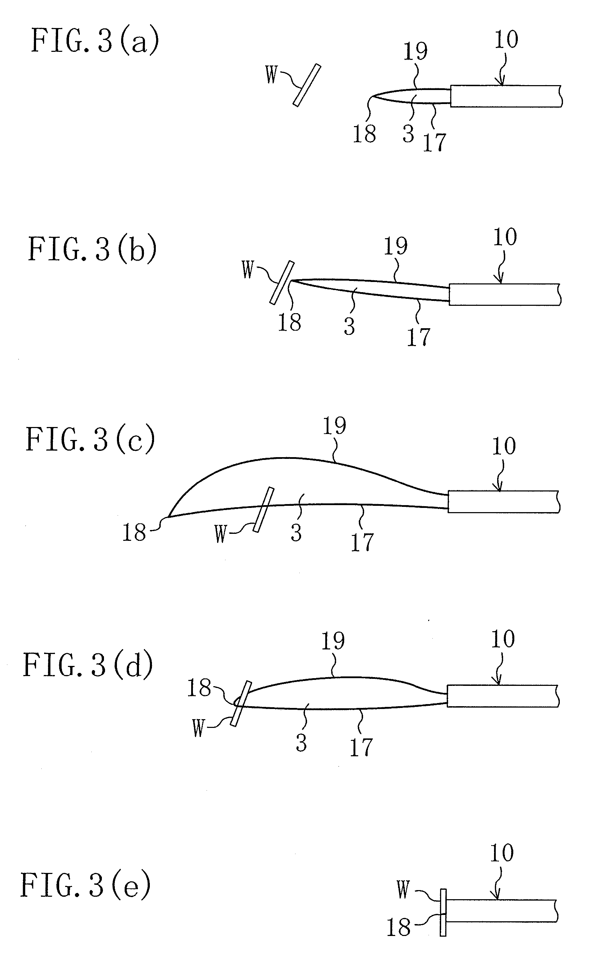

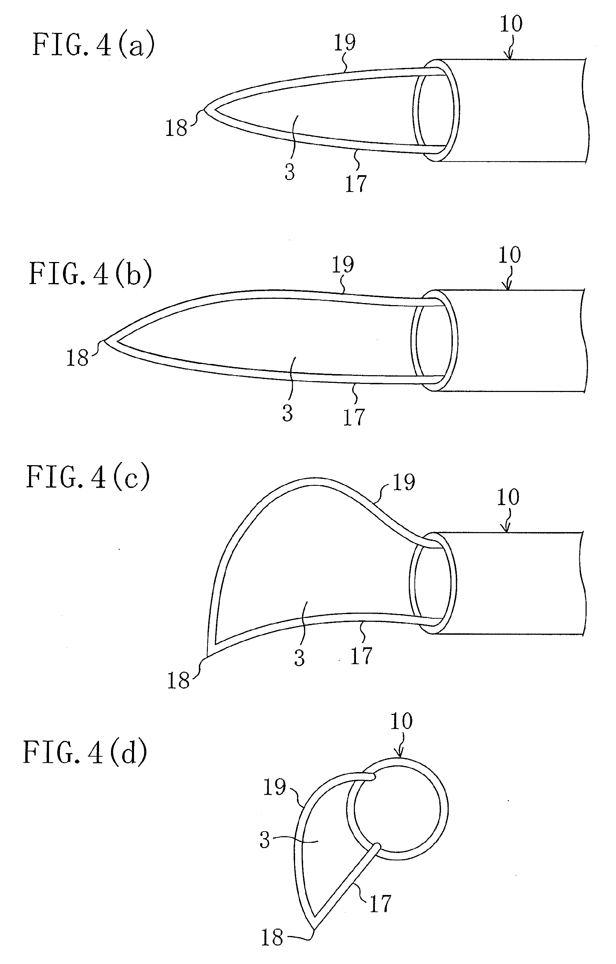

A scheme for using the thus constructed holder **1** will be discussed next with reference to FIG. **3** and FIG. **4**. In the present embodiment, description is given about a scheme for taking out a stent **W** indwelling in a blood vessel (not shown) from the outside of the patient's body by holding it by the holder **1** in the case where the stent **W** falls off from the blood vessel. The stent **W** is the target object. This treatment is carried out under radiography.

First, the first fixing mechanism **30** and the second fixing mechanism **40** release the fixing states. The first operation part **20** and the second operation part **23** are operated to pull to accommodate the end parts on the guide tube **10** side of the first linear portion **17** and the second linear portion **19** and the return portion **18** into the guide tube **10**. The first linear portion **17**, the return portion **18**, and the second linear portion **19** can be accommodated into the guide tube **10** by easy deformation of the flexible holding member **4**.

Next, the distal end of the guide tube **10** is inserted into the patient's blood vessel together with the holding member **4** and is positioned at the vicinity of the stent **W**. Then, when the first linear portion **17**, the second linear portion **19**, and the return portion **18** are protruded from the distal end of the guide tube **10**, the end parts on the guide tube **10** side of the first linear portion **17** and the second linear portion 19 separate from each other by the urging force of the return portion **18** as shown in FIG. **3(a)** and FIG. **4(a)**. Accordingly, the end part on the guide tube **10** side of the first linear portion **17**, the return portion **18**, and the end part on the guide tube **10** side of the second linear portion **19** form the loop **3** for holding the target object.

The size of the loop **3** becomes larger by elongating each protruding length of the first linear portion **17** and the second linear portion **19** from the distal end of the guide tube **10**, as shown in FIG. **4(b)**, while on the other hand becoming smaller by reducing each protruding length thereof. Elongation of each protruding length of the linear portions **17**, **19** from the guide tube **10** separates the distal end of the loop **3** from the distal end of the guide tube **10**, as shown in FIG. **3(b)**, while shortening of each protruding length thereof makes the distal end of the loop **3** to approach the distal end of the guide tube **10**.

When only the second operation part **23** is moved without moving the first operation part **20** to protrude the second linear portion **19** long from the distal end of the guide tube **10**, the second linear portion **19** curves with a point near the return portion **18** as a starting point to separate from the first linear portion **17**, thereby changing the shape of the loop **3**, as shown in FIG. **3(c)** and FIG.**4(c)**, because the linear portions **17**, **19** continue to each other via the return portion **18**. When only the second linear portion **19** is protruded long from the guide tube **10** in this way, the loop is enlarged as well. In reverse, when only the first operation part **20** is moved without moving the second operation part **23** to protrude the first linear portion **17** long from the distal end of the guide tube **10**, the loop **3** changes in shape and is enlarged similarly. In addition, when one of the linear portions is pulled into the guide tube **10** in the state that the first linear portion **17** and the second linear portion **19** are protruded by substantially the same length from the guide tube **10**, the loop **3** changes in its shape and size.

Moreover, when the second operation part **23** is twisted without moving the first operation part **20**, the twisting force thereof is transmitted from the second linear portion **19** to the return portion **18**. By the twisting force, the entire loop **3** is twisted and deformed to be in a three-dimensional shape from a plane shape, as shown in FIG. **4(d)**. Twisting only the first operation part **20** also twists the entire loop **3** similarly to deform the loop **3** three-dimensionally. In addition, twisting the first operation part **20** and the second operation part **23** changes the direction of the loop **3**.

The first linear portion **17** may be fixed to the first pipe **13** by the first fixing mechanism **30** when it is unnecessary to move the fist operation part **20**. As well, the second linear portion **19** may be fixed to the second pipe **14** by the second fixing mechanism **40** when it is unnecessary to move the second operation part **23**. By making the first linear portion **17** and the second linear portion **19** to be fixable to the first pipe **13** and the second pipe **14**, respectively, the shape, the size, and the like of the loop **3** can be changed easily by operating only one of the linear portions.

As described above, operation of the first operation part **20** and/or the second operation part **23** can change the position, the shape, the size, and the direction of the loop **3**. Hence, even in the case where it is difficult to position the distal end part of the guide tube **10** at the vicinity of the stent **W** according to the shape of a blood vessel or the place where the stent **W** falls off or in the case where the shape of the loop **3** is liable to change by the bloodstream in a blood vessel, it becomes easy to allow the loop **3** to approach the stent **W** and to position the stent W within the loop **3**. To do so, as shown in FIG. **3(c)**, the loop **3** is set to be relatively large by protruding the first linear portion **17** and the second linear portion **19** long from the distal end of the guide tube **10**, as shown in FIG. **3(c)**, so that the stent **W** can be positioned within the loop **3** easily.

The operator operates the first operation part **20** and the second operation part **23** with the stent **W** positioned within the loop **3** to pull the first linear portion **17** and the second linear portion **19** into the guide tube **10**. As the first linear portion **17** and the second linear portion **19** are pulled into the guide tube **10**, the size of the lop **3** becomes smaller and the vicinity of the distal end of the loop **3** approaches the stent **W**, as shown in FIG. **3(d)**. Then, the loop **3** draws the stent **W** up to the guide tube **10** and the stent **W** is held at the return portion **18** of the holding member **4**, as shown in FIG. **3(e)**. The guide tube **10** and the holding member **4** are pulled out from the blood vessel with the stent **W** held for taking the stent **W** out from the patient's body.

Thus, in the holder **1** in accordance with the present embodiment, the holding member **4** for holding the stent **W** falling off within the body is composed of the first linear portion **17**, the second linear portion **19**, and the return portion **18** continuing from the linear portions **17**, **19** and the first operation part **20** and the second operation part **23** are provided at the first linear portion **17** and the second linear portion **19**, respectively. Accordingly, even in the case where it is difficult to insert the guide tube **10** up to the vicinity of the stent **W** and in the case where the loop **3** moves or changes in shape by the bloodstream, the stent **W** can be held effortlessly, thereby reducing inversion on the patient.

Further, the branch pipe **11** is formed in a Y-shape of the first pipe **13** and the second pipe **14** and the first linear portion **17** is inserted through the first pipe **13** while the second linear portion **19** is inserted through the second pipe **14**, so that the first operation part **20** and the second operation part **23** separate from each other. This facilitates individual operation of the two operation parts **20**, **23** to thus achieve favorable operability.

The first fixing mechanism **30** fixes the first linear portion **17** to the first pipe **13** to facilitate slight change in position and the like of the loop **3** by operating only the second operation part **23**. Similarly, the second fixing mechanism **40** fixes the second linear portion **19** to the second pipe **14** to facilitate slight change in position and the like of the loop **3** by operating only the first operation part **20**.

The first cylindrical part **21** and the second cylindrical part **24** are provided at the first operation part **20** and the second operation part **23**, respectively, to facilitate identification of the operation parts **20**, **23**, so that the operator can identify the first operation part **20** and the second operation part **23** effortlessly and correctly. This achieves further favorable operability of the holder **1**.

The guide member **2** is divided into the guide tube **10** to be inserted into the patient's body and the branch pipe to be protruded outside the body, and the guide tube **10** is set softer than the branch pipe **11**, with a result that invasion on the patient can be reduced in inserting the guide tube **10** into the body.

The guide tube **10** and the branch pipe **11** of the guide member **2** are detachably connected to each other, so that the guide tube **10** can be exchanged with another one different in shape with the same branch pipe **11** used.

While the present embodiment describes the case for taking the stent **W** out from the body with the use of the holder **1**, the holder **1** of the present invention may be used for taking out from the body a coil-shaped blocking member for blocking a blood vessel, a coil-shaped blocking member for blocking a foraminulum formed in a heart, or the like when the blocking member is displaced from a desired place, for example. Further, the holder **1** of the present invention can be used for taking out from a heart an electrode lead wire (target object) of a pacemaker when the electrode lead wire is cut due to aging. In the case where an indwelling catheter, which is used for generally-called IVH (intravenous hyperalimentation) to a vein near the heart carried out when a patient is in poor nourishment, is broken and cut by a cardiac valve or the like and a chip (target object) thereof remains in the vein, the chip can be taken out with the use of the holder **1** of the present invention. The holder **1** of the present invention can be used for taking out from the organism any of an indweller, a tissue, and the like within an organism besides the aforementioned target objects.

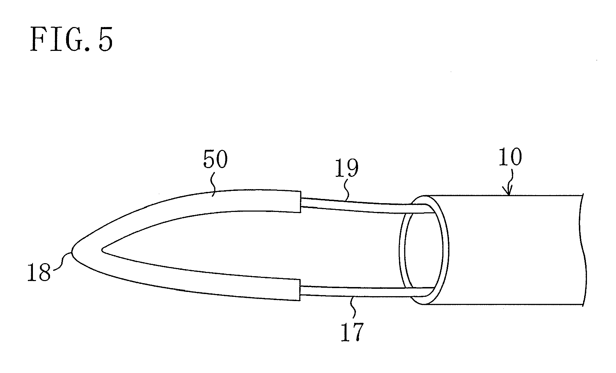

As in Modified Example 1 shown in FIG. **5****,** a non-slip member **50** may be provided at a part of the holding member **4** which forms the distal end part of the loop **3**. The non-slip member **50** is formed of a tubular rubber, for example, for preventing the target object from slipping and falling off from the loop **3**.

The holding member **4** may be formed of, other than a wire, a thin, long, and flexible member capable of transmitting the pushing force in the longitudinal direction thereof when being pushed in the longitudinal direction thereof.

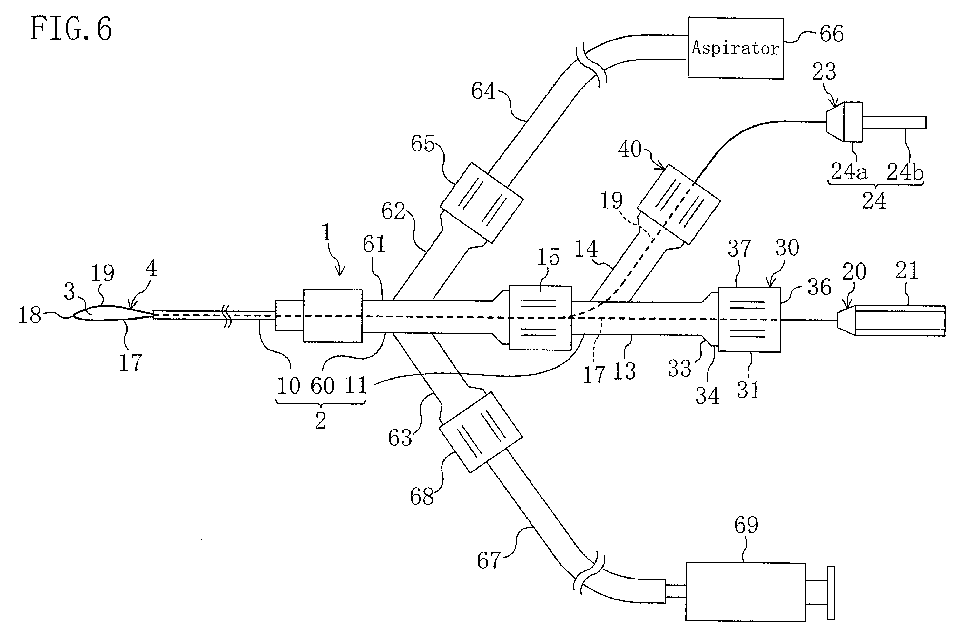

As in Modified Example 2 shown in FIG. **6**, a branch pipe **60** may be provided between the guide tube **10** and the branch pipe **11**. The branch pipe **60** forms a part of the guide member **2** and includes a first pipe **61**, a second pipe **62**, and a third pipe **63**. Each one end of the second pipe **62** and the third pipe **63** communicates with the first pipe **61**. The first pipe **61** communicates with the guide tube **10**, and the holding member **4** is inserted through the first pipe **61**. A joint **65** connected to an aspiration pipe **64** is formed at the other end part of the second pipe **62**. The aspiration pipe **64** is connected to an aspirator **66**. Namely, the guide member **2** is connected to the aspirator **66** through the aspiration pipe **64**. The aspirator **66** is a known tool which is capable of aspirating liquid or air and which is generally used in the medical field and may be of motor-driven type, manually-operated pump type, or the like.

Further, a joint **68** to which an infusion pipe **67** is connected is formed at the other end of the third pipe **63**. The infusion pipe **67** is connected to an insufflator **39**, such as a syringe for infusing a chemical solution. Namely, the guide member **2** is connected to the insufflator **69** through the infusion pipe **67**. Alternatively, only one of the aspiration pipe **64** and the infusion pipe **67** may be connected to the branch pipe **60**. The insufflator **69** may be a motor-driven syringe pump.

According to the holder **1** of Modified Example 2, when the aspirator **66** is operated with the guide tube **10** inserted within an organism, the air in the guide tube **10** can be aspirated. Further, the insufflator **69** can infuse, for example, heparin as an anticoagulant into the guide tube **10** through the infusion pipe **67**, the third pipe **63**, and the first pipe **61**. This prevents the blood from coagulating in the guide tube **10** to maintain the smooth movement of the holding member **4**. An anesthetic for local anesthesia, a physiological salt solution, or the like, for example, may be filled in the insufflator **69** so as to be infused into an organism through the guide tube **10**. The aspirator **66** may aspirate the blood, the anesthetic, and the physiological salt solution, which remain in the guide tube **10**.

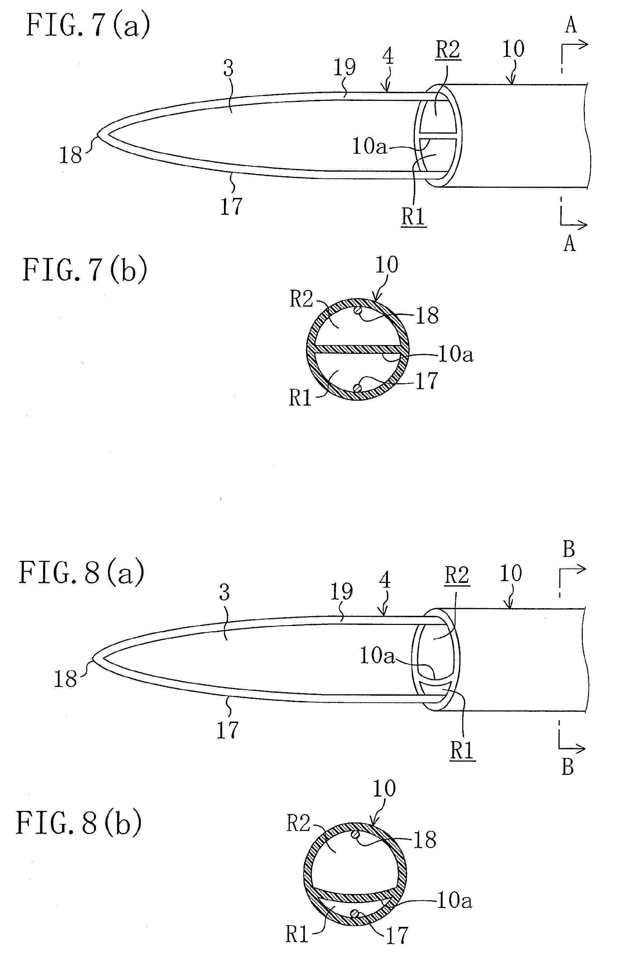

Moreover, as in Modified Example 3 shown in FIG. **7**, a partition wall **10a** for partitioning the inside of the guide tube **10** may be provided inside the guide tube **10** to form a first room **R1** and a second room **R2.** The first linear part **17** is inserted through the first room **R1** while the second linear portion **19** is inserted through the second room **R2**. This prevents, in moving one of the linear portions, the other linear portion from obstructing the one linear portion to facilitate the operation.

In the case where the guide tube **10** is divided into the two rooms **R1, R2,** the second room **R2** may be formed larger than the first room **R1** as in Modified Example 4 shown in FIG. **8**. In this case, the second linear portion **19**, which is inserted through the relatively large second room **R2**, is moved easier than the first linear portion **17**, which is effective in the case where the second linear portion **19** is moved mainly.

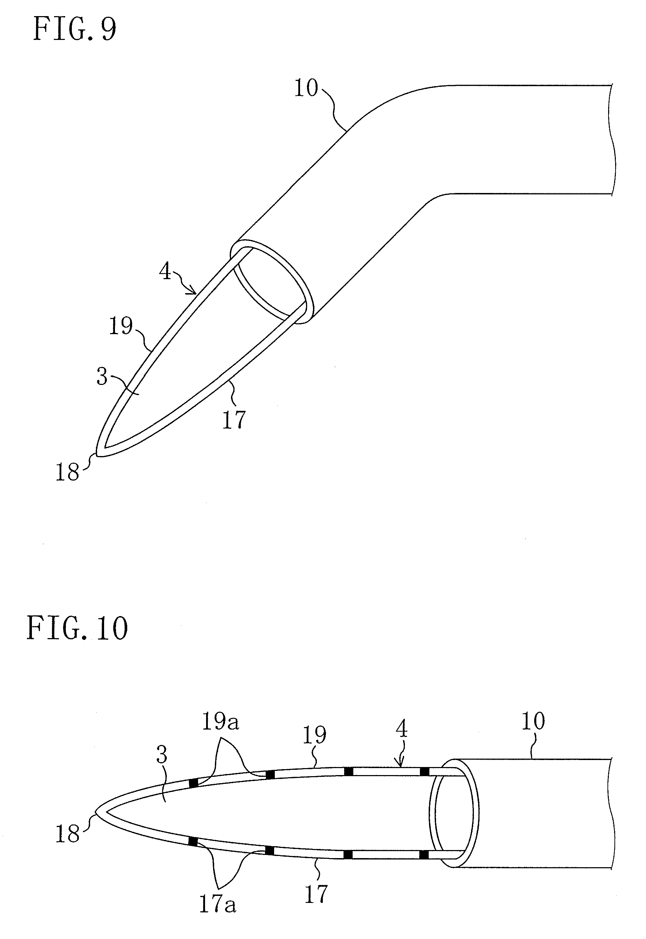

Furthermore, as in Modified Example **5** shown in FIG. **9**, the distal end part of the guide tube **10** may be bent. The bending angle may be set arbitrarily. With this arrangement, only change in direction of the distal end of the guide tube **10** changes the direction that the first linear portion **17** and the second linear portion **19** extend.

In addition, as in Modified Example 6 shown in FIG. **10**, marks **17a**, **19a** may be provided in the first linear portion **17** and the second linear portion **19**, respectively. The marks **17a**, **19a** are made of an X-ray non-transmissive material and are arranged at substantially regular intervals. Accordingly, the operator can recognize the marks **17a**, **19a** under radiography so as to grasp the positions of the linear portions **17**, **19**, the shape and the size of the loop **3**, and the size of the target object **W** with reference to the marks **17a**, **19a**. Each entirety of the first linear portion **17** and the second linear portion **19** may be covered with an X-ray non-transmisive material.

### Industrial Applicability

As described above, the holder in accordance with the present invention can be used for, for example, taking out from the outside of a body a stent or the like falling off from a blood vessel.

## Claims

1. A holder, comprising:
a long tubular guide member to be inserted at one end part thereof into an organism and to be protruded at the other end part thereof from the organism; and
a long holding member of which one end part is inserted in the guide member to be guided into the organism and which forms a loop for holding a target object within the organism,
wherein the holding member includes: a first linear portion extending from the one end part of the holding member and protruded from the other end part of the guide member; a return portion continuing from one end part of the first linear portion and returned toward the other end part of the guide member; and a second linear portion continuing from the return portion and extending toward and protruded from the other end part of the guide member, and
a first operation part and a second operation part which are to be held and operated by an operator are respectively provided at parts of the first linear portion and the second linear portion which are protruded from the other end part of the guide member.

2. The holder of claim 1,
wherein the other end part of the guide member branches into a first pipe through which the other end part of the first linear portion is inserted and a second pipe through which the other end part of the second linear portion is inserted.

3. The holder in claim 2,
wherein the first pipe is provided with first fixing means for switching the first linear portion to and from a state that the other end part thereof is fixed to the first pipe from and to a state that the fixed state is released.

4. The holder of claim 3,
wherein the second pipe is provided with second fixing means for switching the second linear part to and from a state that the other end part thereof is fixed to the second pipe from and to a state that the fixed state is released.

5. The holder of claim 1,
wherein the first operation part and the second operation part are provided with identification parts for identifying the operation parts.

6. The holder of claim 1,
wherein the guide member is divided into an insertion part to be inserted into the organism and a protruded part to be protruded outside the organism, and the insertion part is softer than the protruded part.

7. The holder of claim 1,
wherein an aspirator for aspirating a liquid in the guide member is connected to the guide member.

8. The holder of claim 1,
wherein an insufflator for infusing a chemical solution into the guide member is connected to the guide member.

9. The holder of claim 1,
wherein the guide member is bent at a part on the one end part side thereof.

10. The holder of claim 1,
wherein a mark made of an X-ray non-transmissive material is provided at the one end part of the holding member.

11. The holder of claim 1,
wherein the inside of the guide member is divided into a room through which the first linear portion of the holding member is inserted and a room through which the second linear portion thereof is inserted.
